# EUROPEAN PATENT APPLICATION

(11) **EP 4 354 446 A1**
(43) Date of publication of application: **17.04.2024**
(21) Application number: 22820308.9
(22) Date of filing: 09.06.2022
(51) Int. Cl.: G16B 40/00

(54) **INFORMATION PROCESSING DEVICE, INFORMATION PROCESSING METHOD, AND PROGRAM**

(30) Priority: 09.06.2021 US 202163208509 P
(71) Applicant: Kyoto University, Kyoto-shi, Kyoto 606-8501 (JP); RIKEN, Wako-shi, Saitama 351-0198 (JP)
(72) Inventor: INOUE Haruhisa, Kyoto-shi, Kyoto 606-8501 (JP); YAMAMOTO Takuya, Kyoto-shi, Kyoto 606-8501 (JP); IMAMURA Keiko, Kyoto-shi, Kyoto 606-8501 (JP); KAWAHARA Yoshinobu, Wako-shi, Saitama 351-0198 (JP); UEMATSU Naoya, Wako-shi, Saitama 351-0198 (JP); UEDA Naonori, Wako-shi, Saitama 351-0198 (JP); NAGAHASHI Ayako, Wako-shi, Saitama 351-0198 (JP); ENAMI Takako, Wako-shi, Saitama 351-0198 (JP)
(74) Representative: Cabinet Becker et Associés
(86) International application number: PCT/JP2022/023287
(87) International publication number: WO 2022/260129

(57) **Abstract**

An information processing device includes a processor configured to calculate, for combinations of genes included in a gene data set, a scale of dependence of a multifactorial disease or a sporadic disease on causative genes, and the multifactorial disease or the sporadic disease on related genes, and select a combination including a predetermined number of genes from among the data set based on the scale.

## Description

### [Technical Field]

The present invention relates to an information processing device, an information processing method, and a program.

Priority is claimed on U.S. Provisional Application No. 63/208,509, filed June 9, 2021, the content of which is incorporated herein by reference.

### [Background Art]

Amyotrophic lateral sclerosis (ALS) is a fatal neurodegenerative disease caused by loss of motor neurons, and there is an urgent need to develop diagnostic techniques for ALS.

### [Citation List]

### [Patent Document]

[Patent Document 1]
Japanese Patent Application No. 2017-29116

### [Summary of Invention]

### [Technical Problem]

Since ALS is diagnosed based on clinical findings and electrophysiology studies after clinical symptoms progress, molecular biomarkers for digital diagnosis of ALS are necessary. However, in sporadic ALS, which makes up 90 to 95% of ALS, genes that can serve as molecular biomarkers are still unknown. These challenges are not limited to ALS, but also apply to other multifactorial diseases and sporadic diseases such as Alzheimer's disease and Parkinson's disease which is sporadic in most patients.

An object of the present invention is to provide an information processing device, an information processing method, and a program that can identify genes that can be used to diagnose a multifactorial disease or a sporadic disease.

### [Solution to Problem]

One aspect of the present invention relates to an information processing device including a processor configured to calculate, for combinations of genes included in a gene data set, a scale of dependence of a multifactorial disease or a sporadic disease on causative genes, and the multifactorial disease or the sporadic disease on related genes, and select a combination including a predetermined number of genes from among the data set based on the scale.

### [Advantageous Effects of Invention]

According to one aspect of the present invention, it is possible to identify genes that can be used to diagnose a multifactorial disease or a sporadic disease.

### [Brief Description of Drawings]

FIG. 1 is a diagram illustrating an overview of an embodiment.
FIG. 2 is a diagram showing an example of a configuration of an information processing device according to an embodiment.
FIG. 3 is a flowchart showing a flow of a series of processes performed by a processor for identifying a combination of genes according to an embodiment.
FIG. 4 is a diagram illustrating a method of calculating an HSIC score.
FIG. 5 is a flowchart showing a flow of a series of processes performed by a processor for generating molecular biomarkers for digital diagnosis according to an embodiment.
FIG. 6 is a flowchart showing a flow of a series of processes performed by a processor for determining ALS onset according to an embodiment.
FIG. 7 is a diagram showing a list of ALS causative genes or related genes.
FIG. 8 is a diagram showing calculation results of HSIC scores for combinations of three causative genes.
FIG. 9 is a diagram showing evaluation results of ROC for combinations of causative genes.
FIG. 10 is a diagram showing calculation results of HSIC scores for combinations of three related genes.
FIG. 11 is a diagram showing evaluation results of ROC for combinations of related genes.
FIG. 12 is a list of combinations of genes listed in descending order of HSIC scores.
FIG. 13 is a list of combinations of genes listed in descending order of AUC determined by logistic regression.
FIG. 14 is a diagram showing the top 50 genes with the highest appearance frequency.
FIG. 15 is a diagram showing calculation results of HSIC scores when the number of genes combined is increased to 4.
FIG. 16 is a diagram showing expression levels of PRKAR1A in respective healthy subjects and ALS patients.
FIG. 17 is a diagram showing expression levels of QPCT in respective healthy subjects and ALS patients.
FIG. 18 is a diagram showing expression levels of TMEM71 in respective healthy subjects and ALS patients.
FIG. 19A is a diagram showing a feature space.
FIG. 19B is a diagram showing a feature space.
FIG. 20 is a diagram showing evaluation results of ROC for combinations of PRKAR1A, QPCT, and TMEM71.
FIG. 21A is a diagram showing the correlation between expression amounts of PRKAR1A and survival periods.
FIG. 21B is a diagram showing the correlation between expression amounts of QPCT and survival periods.
FIG. 21C is a diagram showing the correlation between expression amounts of TMEM71 and survival periods.
FIG. 22A is a diagram showing the correlation between expression amounts of PRKAR1A and ages of onset.
FIG. 22B is a diagram showing correlation between expression amounts of QPCT and ages of onset.
FIG. 22C is a diagram showing the correlation between expression amounts of TMEM71 and ages of onset.
FIG. 23A is a diagram showing the correlation between expression amounts of PRKAR1A and a bulbar palsy type and a systemic type.
FIG. 23B is a diagram showing the correlation between expression amounts of QPCT and a bulbar palsy type and a systemic type.
FIG. 23C is a diagram showing the correlation between expression amounts of TMEM71 and a bulbar palsy type and a systemic type.
FIG. 24A is a diagram comparing expression amounts of PRKAR1A in healthy subjects and expression amounts of PRKAR1A in ALS patients.
FIG. 24B is a diagram comparing expression amounts of QPCT in healthy subjects and expression amounts of QPCT in ALS patients.
FIG. 24C is a diagram comparing expression amounts of TMEM71 in healthy subjects and expression amounts of TMEM71 in ALS patients.
FIG. 25 is a diagram showing evaluation results of ROC for combinations of PRKAR1A, QPCT, and TMEM71 extracted from a small number of examples.
FIG. 26 shows an image of iPS cells and a captured image of motor nerve cells obtained from the iPS cells.
FIG. 27A is a diagram comparing expression amounts of PRKAR1A between motor nerve cells of healthy subjects and motor nerve cells of ALS patients.
FIG. 27B is a diagram comparing expression amounts of QPCT between motor nerve cells of healthy subjects and motor nerve cells of ALS patients.
FIG. 27C is a diagram comparing gene expression amounts of TMEM71 between motor nerve cells of healthy subjects and motor nerve cells of ALS patients.
FIG. 28 is a diagram showing evaluation results of ROC for combinations of PRKAR1A, QPCT, and TMEM71 extracted from motor nerve cells.
FIG. 29 is a diagram showing an example of relative expression amounts of TDP-43 for respective genes PRKAR1A, QPCT, and TMEM71.
FIG. 30A is a graph showing expression amounts of PRKAR1A extracted from respective healthy subjects and ALS patients.
FIG. 30B is a graph showing expression amounts of QPCT extracted from respective healthy subjects and ALS patients.
FIG. 30C is a graph showing expression amounts of TMEM71 extracted from respective healthy subjects and ALS patients.
FIG. 31A is a graph showing expression amounts of SPG11 extracted from ALS causative genes and ALS related genes.
FIG. 31B is a graph showing expression amounts of CHMP2B extracted from ALS causative genes and ALS related genes.
FIG. 31C is a graph showing expression amounts of CSNK1G3 extracted from ALS causative genes and ALS related genes.
FIG. 31D is a graph showing expression amounts of DYNC1H1 extracted from ALS causative genes and ALS related genes.

### [Description of Embodiments]

Hereinafter, an information processing device, an information processing method, and a program according to embodiments will be described with reference to the drawings.

### [Overview]

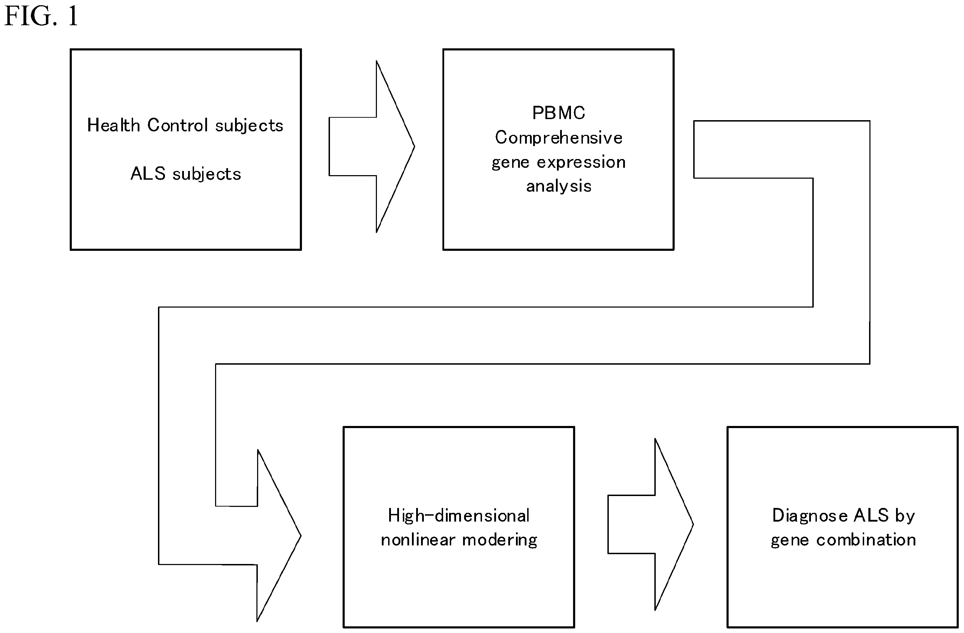

FIG. 1 is a diagram illustrating an overview of the present embodiment. As shown in FIG. 1, in the present embodiment, expression amounts of genes in peripheral blood mononuclear cells (PBMCs) of respective healthy subjects and patients with a multifactorial disease or a sporadic disease are analyzed, and a high-dimensional nonlinear model is used to select gene combinations for classifying healthy subjects and patients with a multifactorial disease or a sporadic disease based on the genes expression amounts. Multifactorial diseases or sporadic diseases include, but are not limited to, for example, ALS, and include Alzheimer's disease and Parkinson's disease. Preferably, sporadic ALS may be exemplified. Hereinafter, as an example, a multifactorial disease or a sporadic disease will be described as "sporadic ALS."

Here, a "multifactorial disease" is defined as a disease that is considered to develop due to the interaction between genetic factors and environmental factors, and a "sporadic disease" is defined as a disease with no recognized family history. However, there are so many cases in which the same disease corresponds to both a "multifactorial disease" and a "sporadic disease" that the "multifactorial disease" and the "sporadic disease" are used almost interchangeably in the field. "Sporadic ALS" is also a multifactorial disease.

### [Configuration of information processing device]

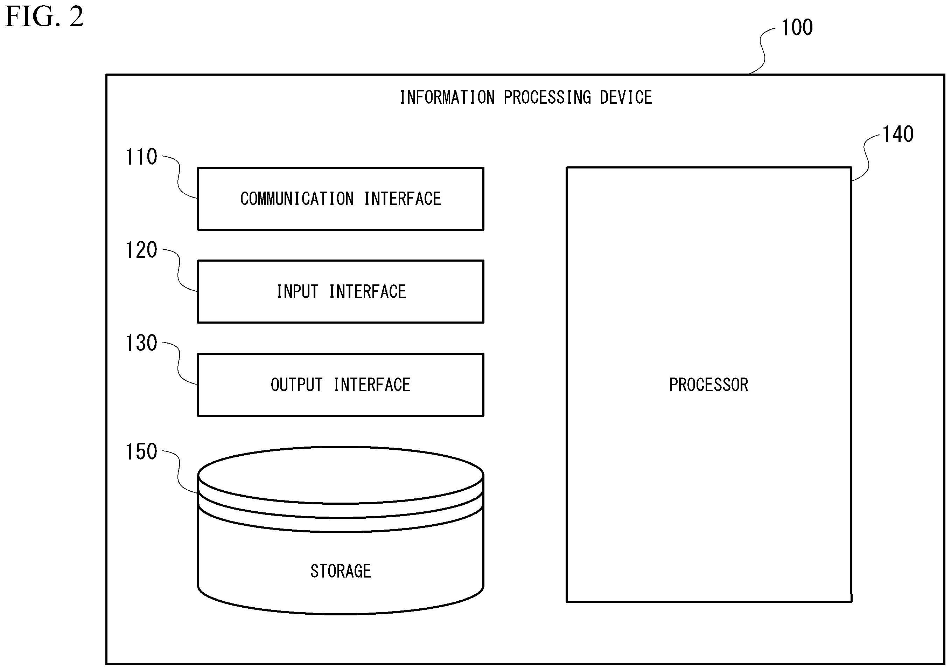

FIG. 2 is a diagram showing an example of a configuration of an information processing device 100 according to an embodiment. As shown, the information processing device 100 includes, for example, a communication interface 110, an input interface 120, an output interface 130, a processor 140, and a storage 150.

The communication interface 110 communicates with an external device via a network, for example, a wide area network (WAN) or a local area network (LAN). For example, the communication interface 110 includes a network interface card (NIC), a wireless communication module and the like. The external device may be, for example, a personal computer or a server installed in facilities where research or drug discovery development is performed (for example, research institutes, universities, and companies).

The input interface 120 receives various input operations from a user, and outputs electrical signals corresponding to the received input operations to the processor 140. For example, the input interface 120 is a mouse, a keyboard, a touch panel, a drag ball, a switch, a button or the like.

The output interface 130 is, for example, a display or a speaker. The display may be, for example, a liquid crystal display (LCD) or an organic electro luminescence (EL) display. The display may be a touch panel formed integrally with the input interface 120.

The processor 140 is realized by, for example, a processor such as a central processing unit (CPU) or a graphics processing unit (GPU) executing a program stored in the storage 150. Some or all of the functions of the processor 140 may be realized by hardware such as a large scale integration (LSI), an application specific integrated circuit (ASIC), or a field-programmable gate array (FPGA), or may be realized in cooperation of software and hardware. Respective functions of the processor 140 will be described below.

The storage 150 is realized by, for example, a hard disc drive (HDD), a flash memory, an electrically erasable programmable read only memory (EEPROM), a read only memory (ROM), or a random access memory (RAM). The storage 150 stores various programs such as a firmware and application programs.

### [Identification of combination of genes]

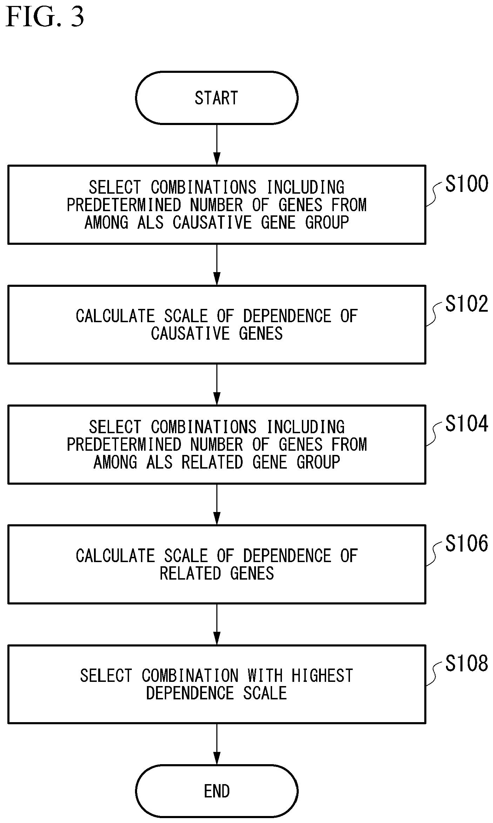

FIG. 3 is a flowchart showing a flow of a series of processes performed by the processor 140 for identifying a combination of genes according to an embodiment.

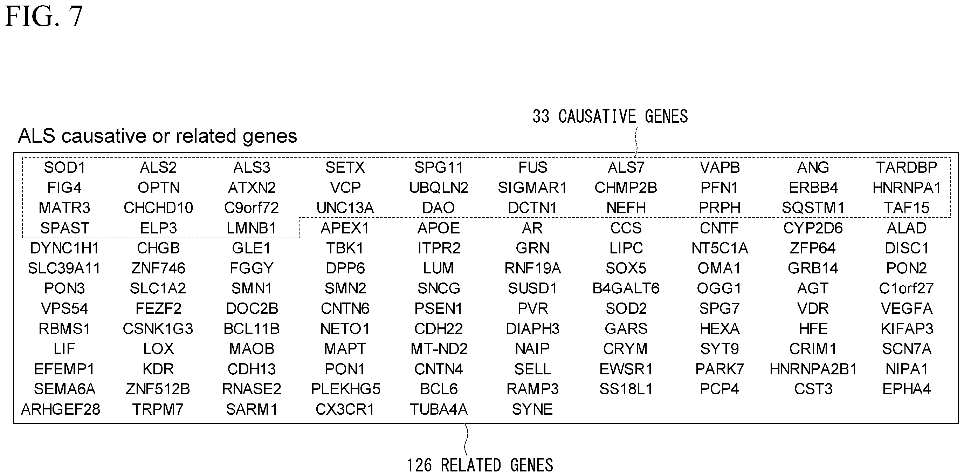

First, the processor 140 selects combinations including a predetermined number of genes from among the ALS causative gene group (Step S100). As ALS causative genes, for example, 33 genes including SOD1, ALS2, ALS3, and SETX are known (refer to FIG. 7 below for details). The processor 140 selects combinations including a predetermined number of genes from among 33 causative genes. The predetermined number is preferably 3, but the number is not limited thereto and it may be, for example, 2 or 4 or more. Hereinafter, as an example, a case in which the predetermined number is "3" will be described. For example, when any three genes are selected from among 33 causative genes, the processor 140 may select 5,456 combinations.

Next, the processor 140 calculates a scale of dependence (or independence) between the causative genes combined in S100 (Step S102).

For gene expression analysis, linear models such as linear logistic regression and Hotelling's t2 test are generally used. However, life phenomena are considered as nonlinear science, and the pathogenesis of diseases cannot be explained by a single factor. Therefore, in the present embodiment, gene expression is analyzed using a nonlinear model.

For example, the processor 140 calculates an HSIC score as a dependence scale of a combination of causative genes using a Hilbert-Schmidt independence criterion (HSIC) which is a type of machine learning and with which a nonlinear structure can be detected in high-dimensional data.

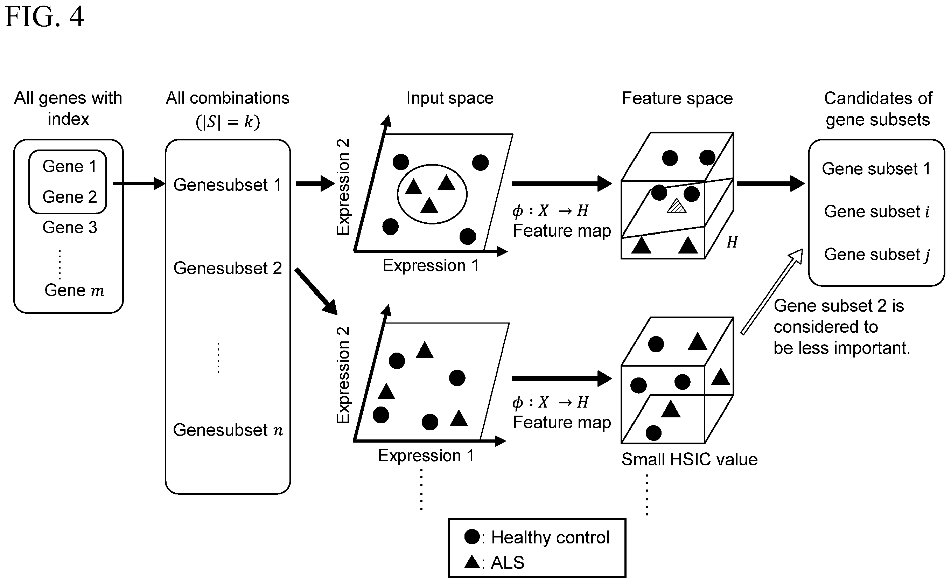

FIG. 4 is a diagram illustrating a method of calculating an HSIC score. As shown, the processor 140 distributes combinations of ALS causative genes (vector data representing respective three causative genes) in a (reproducing kernel) Hilbert space (a feature space in the drawing), and calculates the HSIC score between causative genes on the Hilbert space. For example, the processor 140 calculates HSIC scores for all 5,456 combinations.

Next, the processor 140 selects combinations including a predetermined number of genes from among the ALS related gene group (Step S104). As the ALS related genes, for example, in addition to the above causative genes, additionally, 126 genes such as APEX1, APOE, AR, and CCS are known (refer to FIG. 7 below for details). The processor 140 selects combinations including a predetermined number of genes from among 126 related genes. As described above, the predetermined number is preferably 3, but the number is not limited thereto, and may be, for example, 2 or 4 or more. For example, when any three genes are selected from among 126 related genes, the processor 140 may select 325,500 combinations.

Next, the processor 140 calculates a scale of dependence (or independence) between the related genes combined in S104 (Step S 106). The processor 140 calculates HSIC scores for all 325,500 combinations as in the case of the causative genes.

Next, the processor 140 selects a gene combination with the highest HSIC score from among the gene combinations for which the HSIC score is calculated (Step S108).

For example, in order to eliminate the influence of multicollinearity, the processor 140 performs linear regression analysis such as logistic regression, and selects or extracts a specific combination including genes with a high appearance frequency (number of appearances) from a set of a plurality of combinations for which the HSIC score is calculated (hereinafter referred to as a combination population). For example, the processor 140 may select or extract a combination including genes whose appearance frequency is equal to or more than a threshold value as a specific combination (in other words, a combination to be excluded). The threshold value is, for example, 10, but the value is not limited thereto, and any other value may be used.

The processor 140 excludes the specific combination including genes with a high appearance frequency from the combination population. The processor 140 selects the gene combination with the highest HSIC score from among the combination population from which the specific combination is excluded. As will be described in examples below, as the gene combination with the highest HSIC score, a combination of PRKAR1A, QPCT, and TMEM71 is selected from among the combinations of ALS causative genes or related genes. Accordingly, a series of processes related to identification of gene combinations are completed.

### [Generation of molecular biomarkers for digital diagnosis]

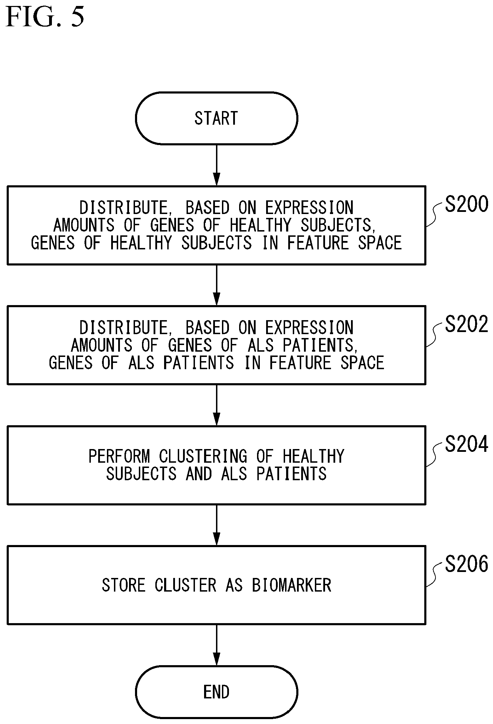

FIG. 5 is a flowchart showing a flow of a series of processes performed by the processor 140 for generating molecular biomarkers for digital diagnosis according to an embodiment.

First, the processor 140 distributes, based on expression amounts of PRKAR1A, QPCT, and TMEM71 of a plurality of respective healthy subjects (hereinafter referred to as a healthy subject group), gene data of the healthy subjects in a three-dimensional feature space whose dimensions are expression amounts of these three genes (Step S200). For example, the gene data of the healthy subjects distributed in the feature space may be represented as a three-dimensional vector (e1, e2, e3) including the expression amount of PRKAR1A as a first element e1, the expression amount of QPCT as a second element e2, and the expression amount of TMEM71 as a third element e3.

Next, the processor 140 distributes, based on expression amounts of PRKAR1A, QPCT, and TMEM71 of a plurality of respective ALS patients (hereinafter referred to as an ALS patient group), gene data of the ALS patients in a three-dimensional feature space whose dimensions are expression amounts of these three genes (Step S202). The gene data of the ALS patients distributed in the feature space may be represented as a three-dimensional vector (e1, e2, e3) like the gene data of healthy subjects.

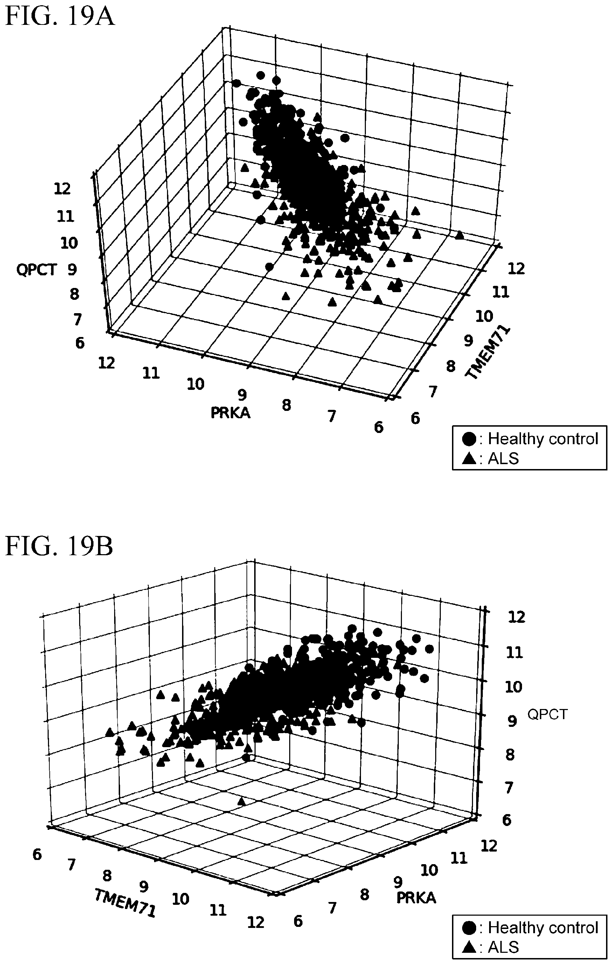

Next, the processor 140 clusters the gene data of healthy subjects and the gene data of ALS patients in the three-dimensional feature space (Step S204). For example, as shown in FIG. 19A and FIG. 19B in examples to be described below, in a three-dimensional feature space whose dimensions are the expression amount of RKAR1A, the expression amount of QPCT, and the expression amount of TMEM71, the processor 140 classifies the gene data of healthy subjects (Health Control in the drawing) and the gene data of ALS patients (ALS in the drawing) into clusters.

Next, the processor 140 stores clusters of gene data of healthy subjects and clusters of gene data of ALS patients formed in the feature space as molecular biomarkers for digital diagnosis in the storage 150 (Step S206). Accordingly, a series of processes related to generation of molecular biomarkers for digital diagnosis are completed.

### [Determination of ALS onset]

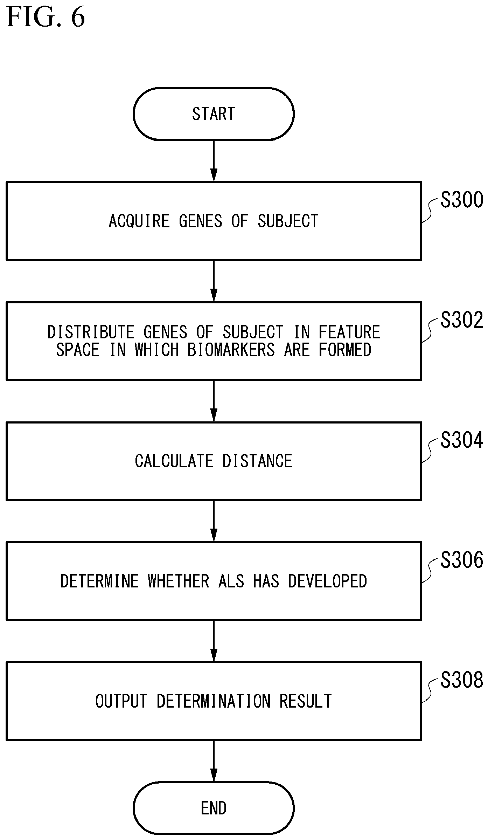

FIG. 6 is a flowchart showing a flow of a series of processes performed by the processor 140 for determining ALS onset according to an embodiment.

First, the processor 140 acquires gene data of a subject for diagnosis of ALS (Step S300). The gene data of the subject may be represented as a three-dimensional vector (e1, e2, e3) in the same manner as above.

Next, the processor 140 distributes the gene data of the subject in a feature space in which clusters (clusters of healthy subjects and clusters of ALS patients) are formed as molecular biomarkers (Step S302).

Next, the processor 140 calculates, on the feature space, a distance D1 between the gene data of the subject and the cluster of the healthy subjects and calculates a distance D2 between the gene data of the subject and the cluster of ALS patients (Step S304).

Next, the processor 140 determines whether the subject will develop ALS at some point in the future or whether the subject has already developed ALS at the present time based on the respective distance of two clusters (Step S306).

For example, when the distance D2 to the cluster of ALS patients is shorter than the distance D1 to the cluster of healthy subjects (D1>D2), that is, when the gene data of the subject is closer to the cluster of ALS patients than the cluster of healthy subjects, the processor 140 may determine that the subject will develop ALS at some point in the future or the subject had already developed ALS at the present time.

On the other hand, when the distance D2 to the cluster of ALS patients is longer than the distance D1 to the cluster of healthy subjects (D1<D2), that is, when the gene data of the subject is closer to the cluster of healthy subjects than the cluster of ALS patients, the processor 140 may determine that the subject will not develop ALS at some point in the future and the subject has not developed ALS at the present time.

Next, the processor 140 outputs the determination result regarding whether the subject has developed ALS (Step S308).

For example, the processor 140 may transmit the determination result to an external device via the communication interface 110 or may output the determination result via the output interface 130 (for example, a display). Accordingly, a series of processes related to the determination of ALS onset are completed.

According to the embodiment described above, the information processing device 100 calculates, for combinations of ALS causative genes or related genes, a scale (for example, HSIC score) of dependence between the genes included in the combinations, and selects a combination with the highest scale from among the plurality of combinations (that is, from among the combination population) for which the scale is calculated. Accordingly, it is possible to identify genes that can be used to diagnose ALS.

In addition, according to the above embodiment, the information processing device 100 distributes the gene data of healthy subjects in the feature space based on expression amounts of the genes derived from the healthy subjects (genes included in the above combination with the highest scale) and additionally, distributes the gene data of ALS patients in the same feature space based on expression amounts of the genes derived from the ALS patients (genes included in the above combination with the highest scale). Then, the information processing device 100 clusters the gene data of healthy subjects and the gene data of ALS patients on the feature space. Accordingly, it is possible to generate molecular biomarkers for digital diagnosis on the feature space.

In addition, according to the above embodiment, the information processing device 100 acquires gene data of a subject for diagnosis of ALS and distributes the gene data of the subject in the feature space in which the clusters of healthy subjects and ALS patients are formed. The information processing device 100 calculates the distance between each cluster and the gene data of the subject on the feature space, and determines, based on the distance, whether the subject will develop ALS at some point in the future or whether the subject has already developed ALS at the present time. Accordingly, it is possible to accurately determine whether ALS has developed.

While forms for implementing the present invention have been described above with reference to embodiments, the present invention is not limited to limited to the embodiments at all, and various modifications and substitutions can be made without departing from the spirit and scope of the present invention.

The above embodiments can be expressed as follows.

### (Appendix 1)

An information processing device, including
a processor configured to calculate, for combinations of genes included in a gene data set, a scale of dependence of a multifactorial disease or a sporadic disease on causative genes, and the multifactorial disease or the sporadic disease on related genes, and select a combination including a predetermined number of genes from among the data set based on the scale.

### (Appendix 2)

The information processing device according to Appendix 2,
wherein the processor distributes, based on an expression amount of a first gene which is a gene derived from a healthy subject, data of the first gene on a certain feature space,
distributes, based on an expression amount of a second gene which is a gene derived from a patient with the multifactorial disease or the sporadic disease, data of the second gene on the feature space,
clusters, based on the expression amount of the first gene, the data of the first gene on the feature space, and
clusters, based on the expression amount of the second gene, the data of the second gene on the feature space.

### (Appendix 3)

The information processing device according to Appendix 2,
wherein the processor distributes, in the feature space in which the clusters of the healthy subject and the patient are formed, data of a third gene which is a gene derived from a subject for diagnosis of the multifactorial disease or the sporadic disease,
calculates a distance between the data of the third gene and the cluster on the feature space, and
based on the distance, determines whether the subject will develop the multifactorial disease or the sporadic disease, or determines whether the subject has developed the multifactorial disease or the sporadic disease.

### (Appendix 4)

The information processing device according to Appendix 1 of 2,
wherein the multifactorial disease or the sporadic disease includes amyotrophic lateral sclerosis,
wherein the predetermined number is 3, and
wherein the combination including the predetermined number of genes includes at least PRKAR1A, QPCT, and TMEM71.

### (Appendix 5)

The information processing device according to Appendix 1 or 2,
wherein the processor
performs linear regression analysis and excludes a specific combination including genes whose appearance frequency is equal to or more than a threshold value from a population which is a set of the gene combinations for which the scale is calculated, and
selects the gene combination with the highest scale from the population from which the specific combination is excluded.

### (Appendix 6)

The information processing device according to Appendix 1 of 2,
wherein the processor
distributes the data set on a Hilbert space,
for the gene combinations included in the data set distributed on the Hilbert space, calculates a Hilbert-Schmidt dependence scale as the scale, and
selects the combination with the highest Hilbert-Schmidt dependence scale from among the plurality of combinations for which the Hilbert-Schmidt dependence scale is calculated.

### (Appendix 7)

An information processing method using a computer, including:
calculating, for combinations of genes included in a gene data set, a scale of dependence of a multifactorial disease or a sporadic disease on causative genes, and the multifactorial disease or the sporadic disease on related genes; and
selecting a combination including a predetermined number of genes from among the data set based on the scale.

### (Appendix 8)

The information processing method according to Appendix 7, further including:
distributing, based on an expression amount of a first gene which is a gene derived from a healthy subject, data of the first gene on a certain feature space;
distributing, based on an expression amount of a second gene which is a gene derived from a patient with the multifactorial disease or the sporadic disease, data of the second gene on the feature space;
clustering, based on the expression amount of the first gene, the data of the first gene on the feature space; and
clustering, based on the expression amount of the second gene, the data of the second gene on the feature space.

### (Appendix 9)

The information processing method according to Appendix 8, further including:
distributing, in the feature space in which the clusters of the healthy subject and the patient are formed, data of a third gene which is a gene derived from a subject for diagnosis of the multifactorial disease or the sporadic disease;
calculating a distance between the data of the third gene and the cluster on the feature space; and
based on the distance, determining whether the subject will develop the multifactorial disease or the sporadic disease, or determining whether the subject has developed the multifactorial disease or the sporadic disease.

### (Appendix 10)

The information processing method according to Appendix 7 or 8,
wherein the multifactorial disease or the sporadic disease includes amyotrophic lateral sclerosis,
wherein the predetermined number is 3, and
wherein the combination including the predetermined number of genes includes at least PRKAR1A, QPCT, and TMEM71.

### (Appendix 11)

The information processing method according to Appendix 7 or 8, further including:
performing linear regression analysis and excluding a specific combination including genes whose appearance frequency is equal to or more than a threshold value from a population which is a set of the gene combinations for which the scale is calculated; and
selecting the gene combination with the highest scale from the population from which the specific combination is excluded.

### (Appendix 12)

A program causing a computer to execute:
calculating, for combinations of genes included in a gene data set, a scale of dependence of a multifactorial disease or a sporadic disease on causative genes, and the multifactorial disease or the sporadic disease on related genes; and
selecting a combination including a predetermined number of genes from among the data set based on the scale.

### (Appendix 13)

The program according to Appendix 12, further including:
distributing, based on an expression amount of a first gene which is a gene derived from a healthy subject, data of the first gene on a certain feature space;
distributing, based on an expression amount of a second gene which is a gene derived from a patient with the multifactorial disease or the sporadic disease, data of the second gene on the feature space;
clustering, based on the expression amount of the first gene, the data of the first gene on the feature space; and
clustering, based on the expression amount of the second gene, the data of the second gene on the feature space.

### (Appendix 14)

The program according to Appendix 13, further including:
distributing, in the feature space in which the clusters of the healthy subject and the patient are formed, data of a third gene which is a gene derived from a subject for diagnosis of the multifactorial disease or the sporadic disease;
calculating a distance between the data of the third gene and the cluster on the feature space; and
based on the distance, determining whether the subject will develop the multifactorial disease or the sporadic disease, or determining whether the subject has developed the multifactorial disease or the sporadic disease.

### (Appendix 15)

The program according to Appendix 12 or 13,
wherein the multifactorial disease or the sporadic disease includes amyotrophic lateral sclerosis,
wherein the predetermined number is 3, and
wherein the combination including the predetermined number of genes includes at least PRKAR 1A, QPCT, and TMEM71.

### (Appendix 16)

The program according to Appendix 12 or 13, further including:
performing linear regression analysis and excluding a specific combination including genes whose appearance frequency is equal to or more than a threshold value from a population which is a set of the gene combinations for which the scale is calculated; and
selecting the gene combination with the highest scale from the population from which the specific combination is excluded.

### (Appendix 17)

A computer-readable storage medium in which the program according to Appendix 12 or 13 is stored.

### [Examples]

### [Experiment Example 1]

### (Microarray data and normalization)

Gene expression data (GSE112676, 233 ALS and 508 CTL) was used for HSIC analysis. Gene expression signals were normalized by downloading raw expression intensities and detected p-values (GSE112676_HT12_V3_preQC_nonnormalized.txt) and using R limma package (v3. 32.10) function (background Correct and normalize Between Arrays). In order to remove a batch effect, the ComBat algorithm implemented in the R sva package (v3. 35.2) was used. One sample (GSM3077426) with an abnormal value even after batch effect correction was excluded from further analysis. For the above HSIC prediction in FIG. 1, ALS related genes in ALS online database (ALSoD, https://alsod.ac.uk/) were used. Unbiased HSIC prediction was performed using the top 1,000 variable genes among the detectably expressed genes of 20% or more of samples.

### [Experiment Example 2]

### (Preparation of pluripotent stem cells)

Pluripotent stem cells were prepared. Examples of pluripotent stem cells included embryonic stem cells (ES cells), induced pluripotent stem cells (iPS cells), embryonic stem (ntES) cells derived from cloned embryos obtained by nuclear transplantation, sperm stem cells (GS cells), and embryonic germ cells (EG cells). Preferable pluripotent stem cells were ES cells, iPS cells or ntES cells. More preferable pluripotent stem cells were human pluripotent stem cells, and human ES cells and human iPS cells were particularly preferable. In addition, the cells that could be used in the present invention were not only pluripotent stem cells but also cell groups induced by socalled "direct reprogramming," which were induced to directly differentiate into desired cells without passing through pluripotent stem cells. In this experiment, human iPS cells were used. Hereinafter, unless otherwise specified, iPS cells were human iPS cells.

iPS cells were prepared from fibroblasts or PBMCs from healthy subjects and sporadic ALS patients using OCT3/4, Sox2, Klf4, L-Myc, Lin28 and dominant negative p53 episomal vectors, or OCT3/4, Sox2, Klf4, L-Myc, Lin28 and shRNA for p53. The cells were cultured in a feeder-free and xeno-free culture system using StemFit (Ajinomoto) to which penicillin/streptomycin were added.

### [Experiment Example 3]

### (Motor neuron differentiation from iPS cells)

Motor neurons were differentiated from iPS cells. Specifically, the iPS cells were dissociated into single cells, and rapidly reaggregated in a low-cell adhesive U-shaped 96-well plate (Lipidule-Coated Plate A-U96, NOF Corporation, Tokyo, Japan).

Aggregates were treated using 5% KSR (Invitrogen, Waltham, MA), minimum essential medium-non-essential amino acid (Invitrogen), L-glutamine (Sigma-Aldrich, St. Louis, MO), 2-mercaptoethanol (Wako, Osaka, Japan), 2 µM dorsomorphin (Sigma-Aldrich), 10 µM SB431542 (Cayman, Ann Arbor, MI), 3 µM CHIR99021 (Cayman), and 12.5 ng/mL fibroblast growth factor (Wako) in a nerve induction stage for 11 days.

On the 4^{th} day, 100 nM retinoic acid (Sigma-Aldrich) and 500 nM Smoothened ligand (Enzo Life Sciences, Farmingdale, NY) were added. After patterning with a neurobase medium to which B27 Supplement (Thermo Fisher Scientific), 100 nM retinoic acid, 500 nM Smoothened ligand, and 10 µM DAPT (Selleck, Houston, TX) were added, on the 16^{th} day, aggregates were separated using Accumax (Innovative Cell Technologies, San Diego, CA) and dissociated into single cells, and adhered to a dish coated with matrigel (BD Biosciences, Franklin Lakes, NJ).

The adhered cells were cultured in a neural base medium containing 10 ng/ml brain-derived neurotrophic factor (R&D Systems, Minneapolis, MN), 10 ng/ml glial cell line-derived neurotrophic factor (R&D Systems), and 10 ng/ml neurotrophin-3 (R&D Systems) for 8 days. On the 21^{st} day, the cells were dissociated into single cells using Accumax and seeded in an iMatrix-coated 24-well plate (Corning) at 2×105 cells/well.

### [Experiment Example 4]

### (Quantitative RT-PCR)

Total RNA of the cultured cells was extracted using RNeasy Plus Mini kit (QIAGEN). 1 µg of RNA was reversely transcribed using ReverTra Ace (TOYOBO, Osaka, Japan). Quantitative PCR analysis was performed using SYBR Premix Ex TaqII (TAKARA) according to a reverse transcription reaction using StepOnePlus (Thermo Fisher Scientific).

### [Experiment Example 5]

### (Statistical analysis)

The results were analyzed using Student's t-test, and the statistical significance was determined. A difference of p<0.05 was considered significant. The analysis was performed using GraphPad Prism software version 8.0 for Windows (GraphPad Software, SanDiego, CA).

### (Results)

### [Experiment Example 6]

Gene combinations for classifying healthy subjects and ALS patients were selected by analyzing gene expression amounts of peripheral blood mononuclear cells (PBMCs). As described in the above embodiment, the gene expression amounts were analyzed using a nonlinear model, and HSIC was used for the analysis.

Gene combinations with differences between healthy subjects and ALS patients had a high HSIC score, and on the other hand, gene combinations with no differences had an HSIC score close to 0. When a combination with a high HSIC score was identified, genes for classifying healthy subjects and ALS patients were extracted.

First, the validity of the method described in the present embodiment was verified using a gene group known to be associated with ALS. FIG. 7 is a diagram showing a list of ALS causative genes or related genes. For example, from the genes shown in FIG. 7, 33 causative genes known as ALS causative gene were selected, and additionally, combinations of three genes were selected from among the 33 causative genes.

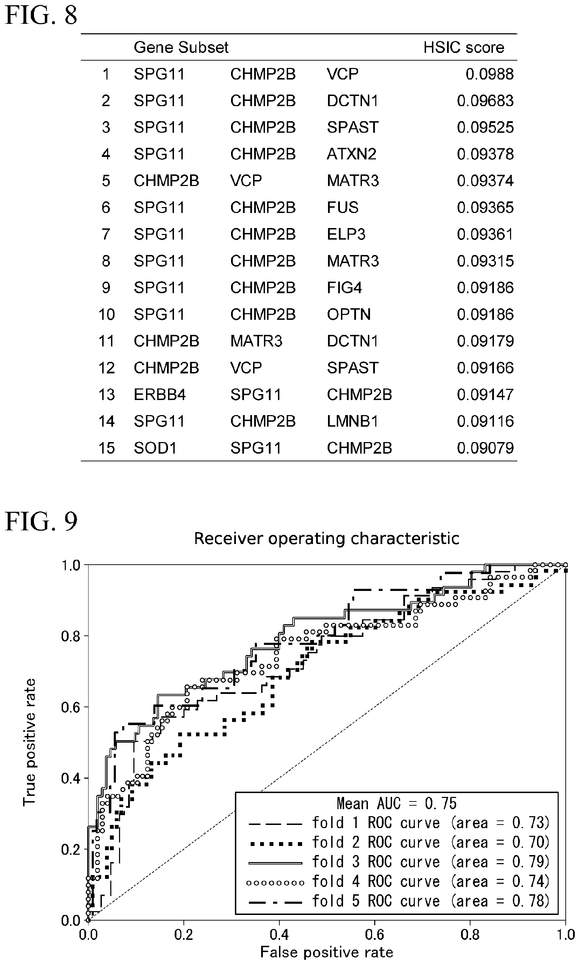

The HSIC score was calculated as a scale for classifying the healthy subject group and the ALS patient group based on expression amounts of the three genes. FIG. 8 is a diagram showing calculation results of HSIC scores for combinations of three causative genes. FIG. 8 shows only the top 15 combinations with the highest HSIC score. The causative gene combination with the highest HSIC score included SPG11, CHMP2B, and VCP (an HSIC score of 0.0988).

Among all combinations (5,456 combinations) of 33 ALS causative genes, the causative gene combination with the highest HSIC score included SPG11, CHMP2B, and VCP (an HSIC score of 0.0988). The combination of these three causative genes was evaluated using Receiver Operating Characteristics (ROC). FIG. 9 is a diagram showing evaluation results of ROC for combinations of causative genes. As shown in FIG. 9, the combination of SPG1 1, CHMP2B, and VCP with the highest HSIC score had an AUC (area under the curve) of 0.75 in ROC. Therefore, in the result, there was a statistically significant difference in AUC.

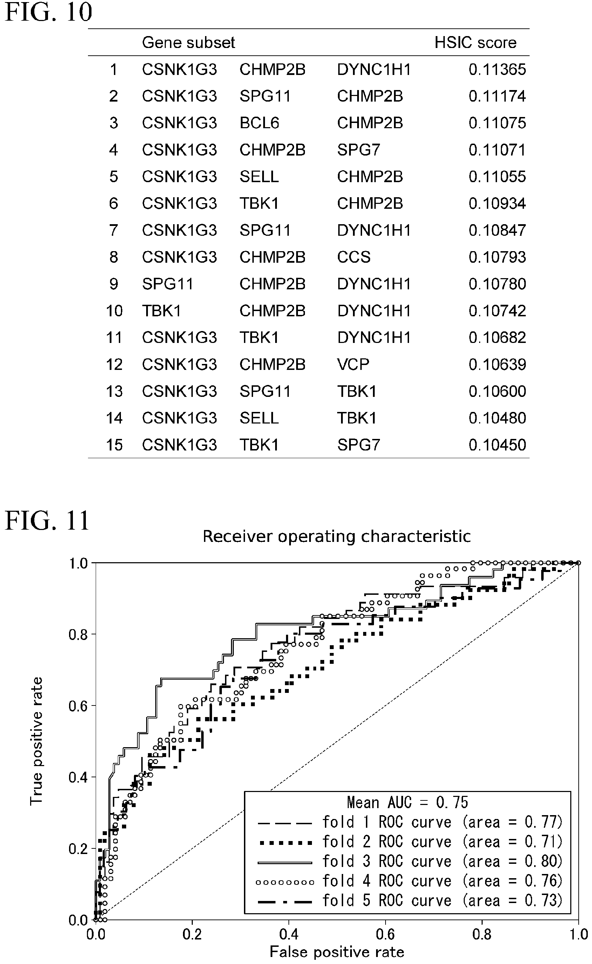

Next, a combination of three genes was similarly selected from among 126 ALS related genes (refer to FIG. 7). In the healthy subject group, the HSIC score of the combination of three related genes was calculated, and in the ALS patient group, the HSIC score of the combination of three related genes was calculated. FIG. 10 is a diagram showing calculation results of HSIC scores for combinations of three related genes. Like FIG. 8, FIG. 10 shows only the top 15 combinations with the highest HSIC score. The related gene combination with the highest HSIC score included CSNK1G3, CHMP2B, and DYNC1H1 (an HSIC score of 0.11365).

Among all combinations (325,500 combinations) of 126 related genes, the related gene combination with the highest HSIC score included CSNK1G3, CHMP2B, and DYNC1H1 (an HSIC score of 0.11365). The combination of these three related genes was evaluated using ROC. FIG. 11 is a diagram showing evaluation results of ROC for combinations of related genes. As shown in FIG. 11, the combination of CSNK1G3, CHMP2B, and DYNC1H1 with the highest HSIC score had an AUC (AUC for classifying the healthy subject group and the ALS patient group) of 0.75 in ROC. Therefore, in the result, there was a statistically significant difference in AUC.

According to these results, the validity of the method of the present embodiment for finding a gene set for classifying the healthy subject group and the ALS patient group was demonstrated.

### [Experiment Example 7]

In order to examine unknown factors of ALS, the HSIC scores of gene combinations among genes (unrelated genes) not known to be associated with ALS were calculated. In order to avoid multicollinearity, which causes a problem in the multivariate regression model, analysis was performed using linear regression, and genes that repeatedly appeared (genes with a high appearance frequency) in the list of genes extracted through the analysis were excluded from the ALS causative genes or related genes.

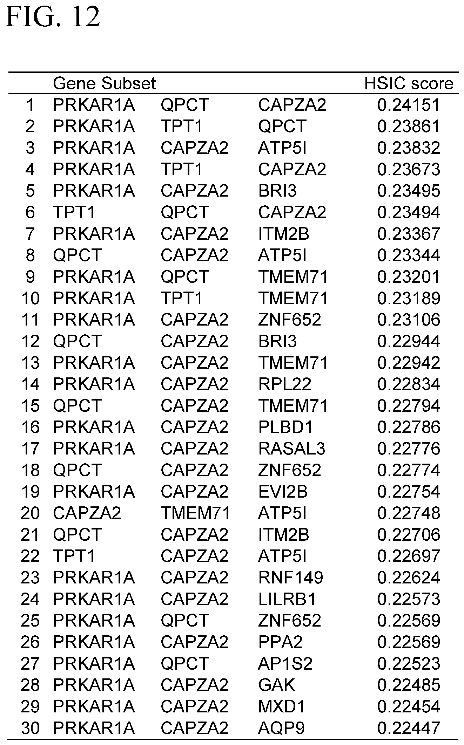

On the other hand, combinations of genes that distinguish between healthy subjects and ALS patients were listed up using logistic regression, which is a linear regression model. FIG. 12 is a list of combinations of genes listed in descending order of HSIC scores.

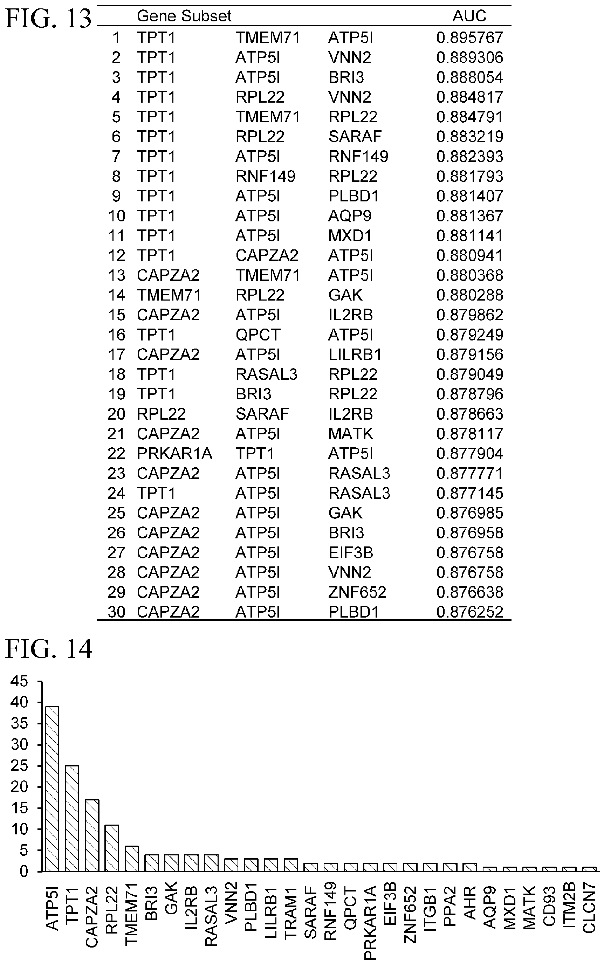

When frequently appearing genes were examined using logistic regression, it was found that there was a bias in the gene appearance frequency. FIG. 13 is a list of combinations of genes listed in descending order of AUC determined by logistic regression. FIG. 14 is a diagram showing the top 50 genes with the highest appearance frequency. As shown in FIG. 14, TPT1 appeared 25 times, ATP5I appeared 39 times, CAPZA2 appeared 17 times, and RPL22 appeared 11 times.

In order to eliminate the influence of multicollinearity, among the gene combinations with a high HSIC score, genes that repeatedly appeared 10 times or more in the linear regression were excluded. In the results of FIG. 14, since TPT1, ATP5I, CAPZA2, and RPL22 all appeared 10 times or more, when combinations including any one of these four genes were excluded, the gene combination with the highest HSIC score included PRKAR1A, QPCT, and TMEM71 (the 9^{th} combination from the top in FIG. 12).

### [Experiment Example 8]

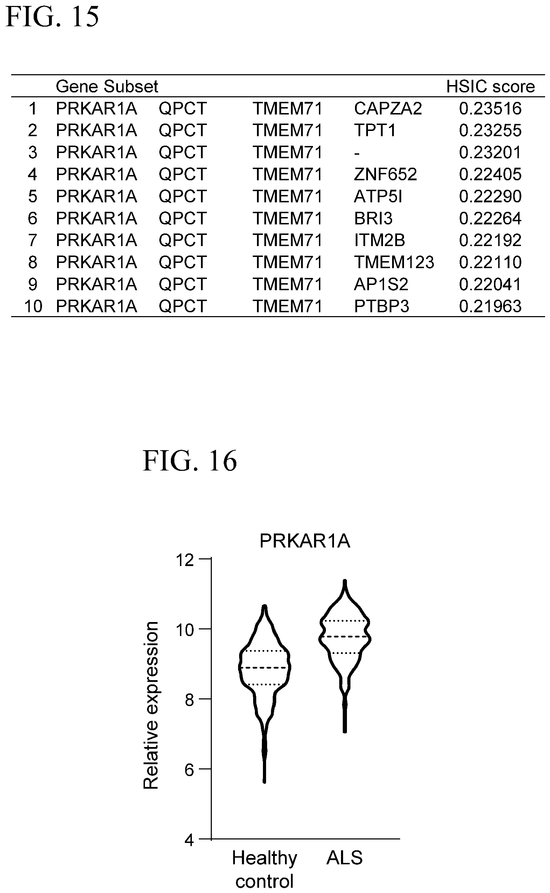

In addition, it was examined whether the ALS classification accuracy increased when the number of genes in the combination was increased to 4 (the predetermined number was changed from 3 to 4). FIG. 15 is a diagram showing calculation results of HSIC scores when the number of genes combined is increased to 4. When four genes were combined, the HSIC score did not change significantly compared to when three genes were combined. Therefore, a combination of three genes was selected.

### [Experiment Example 9]

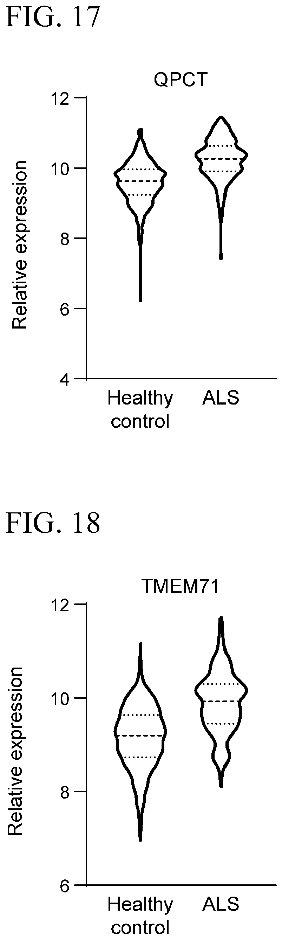

Next, expression levels of PRKAR1A, QPCT, and TMEM71 in PBMCs of healthy subjects and ALS patients were compared. FIG. 16 is a diagram showing expression levels of PRKAR1A in respective healthy subjects and ALS patients. FIG. 17 is a diagram showing expression levels of QPCT in respective healthy subjects and ALS patients. FIG. 18 is a diagram showing expression levels of TMEM71 in respective healthy subjects and ALS patients. The expression levels of all three genes were higher in the ALS patients than in the healthy subjects.

In addition, in a three-dimensional feature space whose dimensions were expression amounts of respective PRKAR1A, QPCT, and TMEM71, the genes of the healthy subjects and the genes of the ALS patients were distributed separately. FIG. 19A and FIG. 19B are diagrams showing a feature space. As shown in FIG. 19A and FIG. 19B, on the three-dimensional feature space, the genes of the healthy subjects were classified into the same cluster, and the genes of the ALS patients were classified into the same cluster.

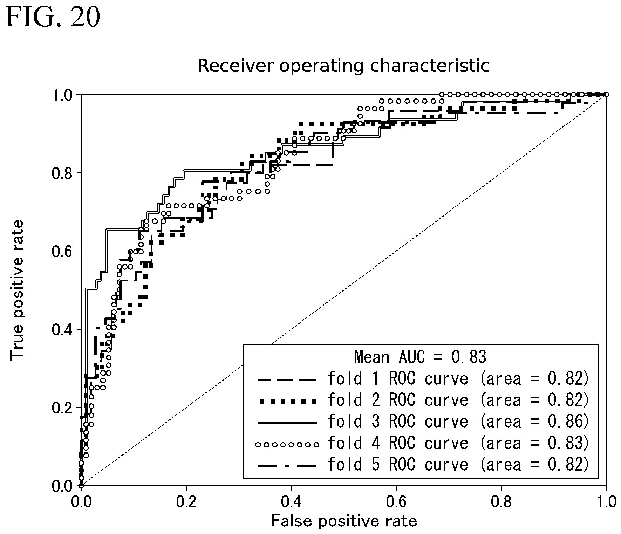

The combination of PRKAR1A, QPCT, and TMEM71 was evaluated using ROC. FIG. 20 is a diagram showing evaluation results of ROC for combinations of PRKAR1A, QPCT, and TMEM71. As shown in FIG. 20, the combination of PRKAR1A, QPCT, and TMEM71 had an AUC (AUC for classifying the healthy subject group and the ALS patient group) of 0.83 in ROC. Therefore, in the result, there was a statistically significant difference in AUC.

### [Experiment Example 10]

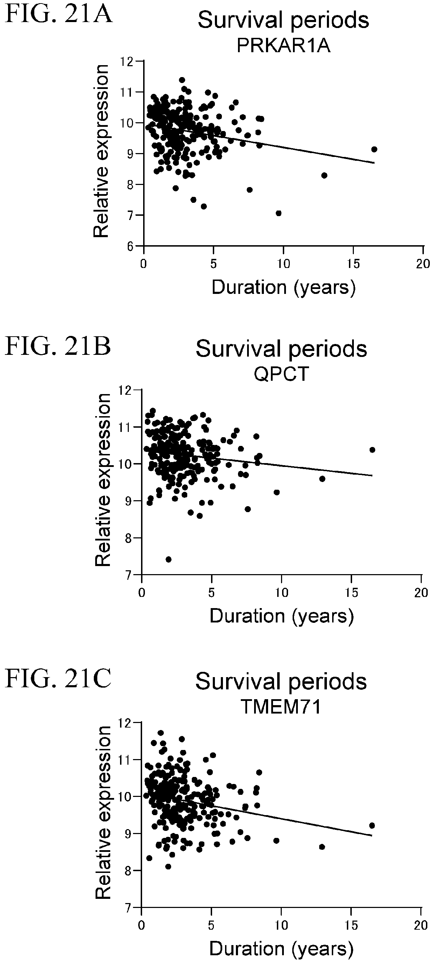

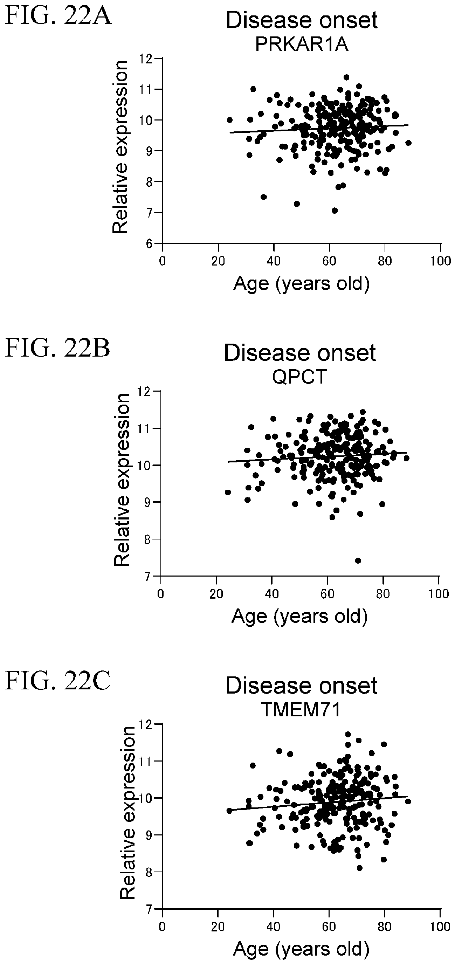

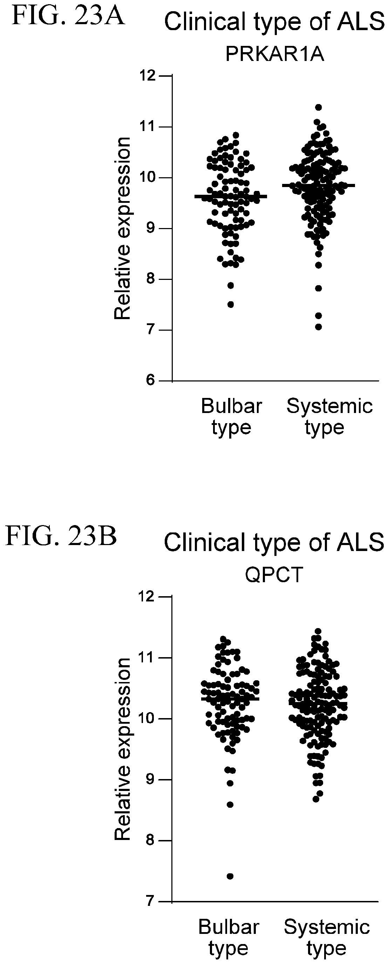

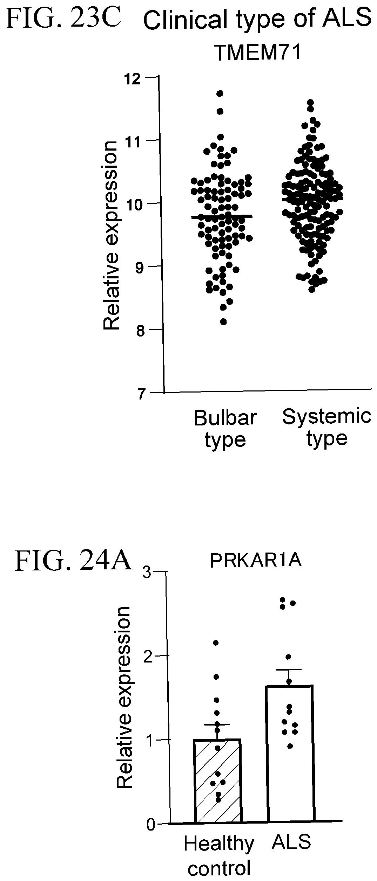

In addition, the relationship between the expression levels of genes PRKAR1A, QPCT, and TMEM71 and clinical information on ALS obtained from published data was examined. FIG. 21A is a diagram showing the correlation between expression amounts of PRKAR1A and survival periods. FIG. 21B is a diagram showing the correlation between expression amounts of QPCT and survival periods. FIG. 21C is a diagram showing the correlation between expression amounts of TMEM71 and survival periods. FIG. 22A is a diagram showing the correlation between expression amounts of PRKAR1A and ages of onset. FIG. 22B is a diagram showing correlation between expression amounts of QPCT and ages of onset. FIG. 22C is a diagram showing the correlation between expression amounts of TMEM71 and ages of onset. FIG. 23A is a diagram showing the correlation between expression amounts of PRKAR1A and a bulbar palsy type and a systemic type. FIG. 23B is a diagram showing the correlation between expression amounts of QPCT and a bulbar palsy type and a systemic type. FIG. 23C is a diagram showing the correlation between expression amounts of TMEM71 and a bulbar palsy type and a systemic type.

As shown in FIGS. 21A, 21B, and 21C, the gene expression amounts of PRKAR1A and TMEM71 did not have a significant correlation in QPCT, but they had a correlation in the survival period. As shown in FIGS. 22A, 22B, and 22C, there was no correlation between the age of onset and the expression amounts of the three genes. As shown in FIGS. 23A, 23B, and 23C, there was no difference in QPCT, but systemic type ALS patients showed significantly higher expression amounts of PRKAR1A and TMEM71 than bulbar palsy type ALS patients.

### [Experiment Example 11]

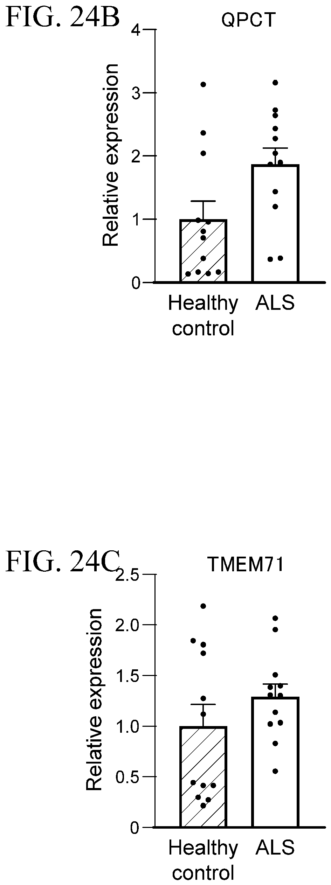

In addition, the expression amounts of the three genes PRKAR1A, QPCT, and TMEM71 were confirmed using PBMCs of healthy subjects and PBMCs of ALS patients that we had. PBMCs were collected from 12 ALS patients and 12 healthy subjects, and RNA was extracted. FIG. 24A is a diagram comparing expression amounts of PRKAR1A in healthy subjects and expression amounts of PRKAR1A in ALS patients. FIG. 24B is a diagram comparing expression amounts of QPCT in healthy subjects and expression amounts of QPCT in ALS patients. FIG. 24C is a diagram comparing expression amounts of TMEM71 in healthy subjects and expression amounts of TMEM71 in ALS patients. As shown in FIGS. 24A, 24B, and 24C, it was observed that the expression amounts of PRKAR1A and QPCT were significantly higher in the ALS patients than in the healthy subjects, and the expression amount of TMEM71 tended to be higher in the ALS patients.

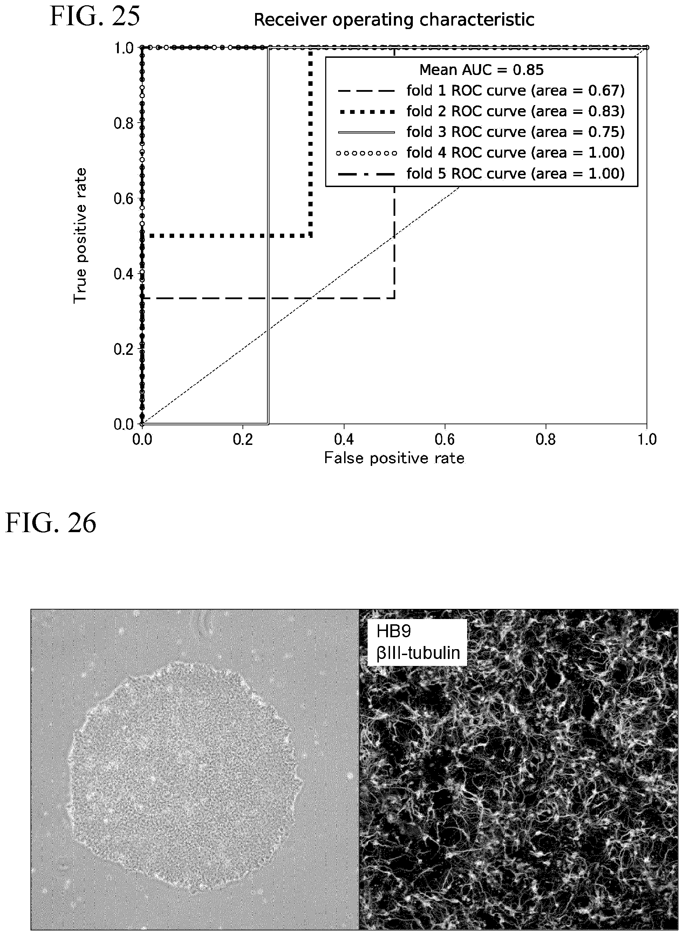

FIG. 25 is a diagram showing evaluation results of ROC for combinations of PRKAR1A, QPCT, and TMEM71 extracted from a small number of examples. As shown in FIG. 25, when the expression amounts of the three genes PRKAR1A, QPCT, and TMEM71 were combined, even with a small number of examples of 12 ALS patients and 12 healthy subjects, the AUC for classifying the healthy subjects and the ALS patients was 0.85. Based on these results, it was confirmed that, when the expression amounts of the three genes in the PBMCs were examined, it was possible to distinguish the ALS patient group and the healthy subject group.

### [Experiment Example 12]

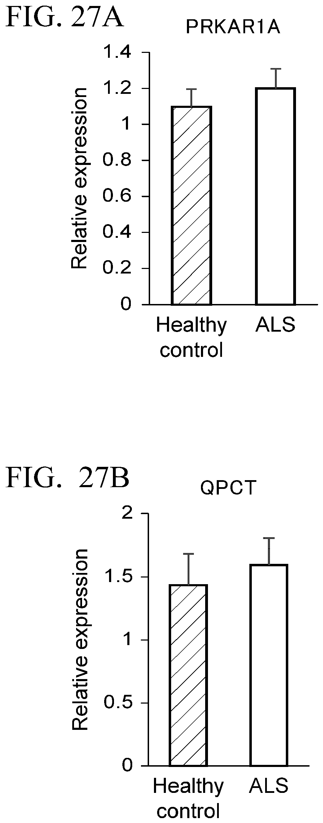

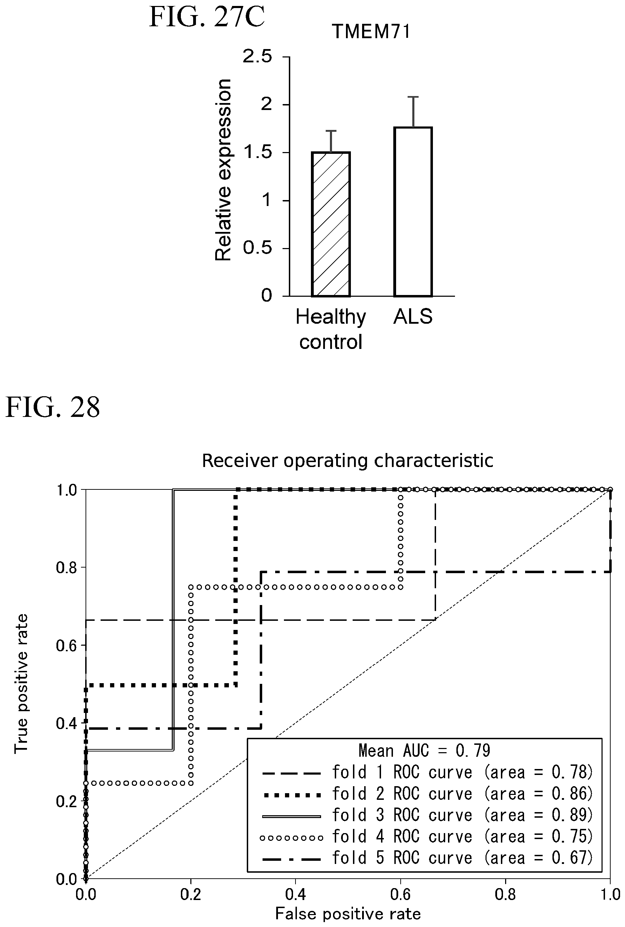

Expression of three genes in motor nerve cells obtained from iPS cells established from 26 healthy subjects and 18 ALS patients was examined. FIG. 26 shows an image of iPS cells and a captured image of motor nerve cells obtained from the iPS cells. FIG. 27A is a diagram comparing expression amounts of PRKAR1A between motor nerve cells of healthy subjects and motor nerve cells of ALS patients. FIG. 27B is a diagram comparing expression amounts of QPCT between motor nerve cells of healthy subjects and motor nerve cells of ALS patients. FIG. 27C is a diagram comparing expression amounts of TMEM71 between motor nerve cells of healthy subjects and motor nerve cells of ALS patients. FIG. 28 is a diagram showing evaluation results of ROC for combinations of PRKAR1A, QPCT, and TMEM71 extracted from motor nerve cells. As shown in FIGS. 27A, 27B, and 27C, there was no difference in the expression amount of each of genes PRKAR1A, QPCT, and TMEM71 between motor nerve cells of healthy subjects and motor nerve cells of ALS patients. As shown in FIG. 28, when the expression amounts of the three genes were compared as a set, the AUC was 0.79 and it was possible to classify the healthy subjects and ALS patients.

### [Experiment Example 13]

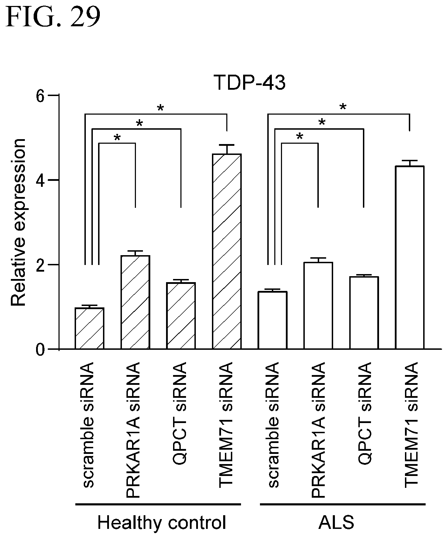

In addition, since accumulation of TDP-43 was deeply involved in the pathogenesis of ALS, the relationship between the three genes and TDP-43 was examined. FIG. 29 is a diagram showing an example of relative expression amounts of TDP-43 for respective genes PRKAR1A, QPCT, and TMEM71. As shown in FIG. 29, when PRKAR1A, QPCT, and TMEM71 were knock-downed with siRNA, the expression amount of TDP-43 significantly increased in motor nerve cells derived from both healthy subjects and ALS patients. Based on these results, although the relationship of the gene group identified using the HSIC in the method of the present embodiment with the pathogenesis of ALS is still not known, a possibility of a new player in the pathogenesis of ALS was found according to this experiment.

### [Experiment Example 14]

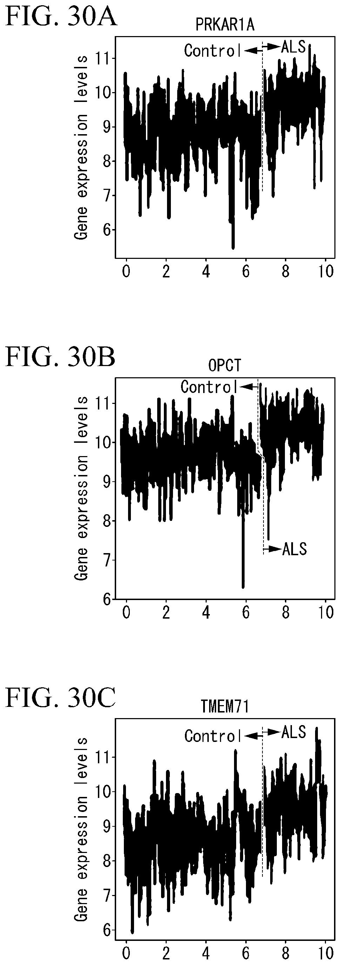

The expression amounts of the three genes extracted from respective healthy subjects and ALS patients were shown in a graph. FIG. 30A is a graph showing expression amounts of PRKAR1A extracted from respective healthy subjects and ALS patients. FIG. 30B is a graph showing expression amounts of QPCT extracted from respective healthy subjects and ALS patients. FIG. 30C is a graph showing expression amounts of TMEM71 extracted from respective healthy subjects and ALS patients. In the drawings, the vertical axis represents gene expression amount, and the horizontal axis represents case.

In each of PRKAR1A, QPCT, and TMEM71, the expression amount of ALS patients tended to be higher than that of healthy subjects, but there was a large variation between samples, and the expression amount of each gene alone could not be used to classify healthy subjects and ALS.

On the other hand, as described in the above embodiment, when the expression amounts of these three genes PRKAR1A, QPCT, and TMEM71 were combined, it was possible to classify the healthy subjects and the ALS patients. Therefore, the usefulness of the combination of three genes extracted by the HSIC was demonstrated.

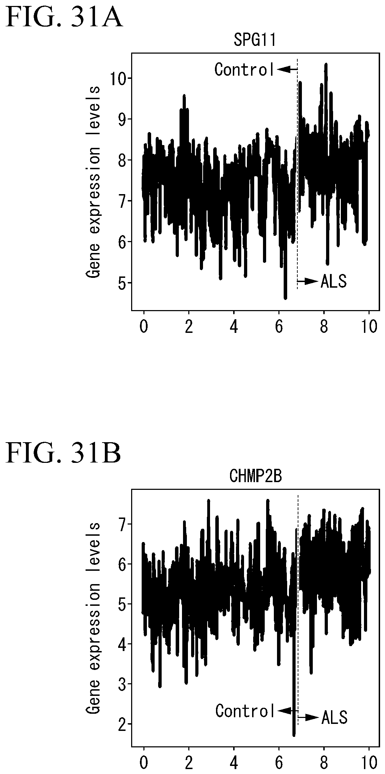

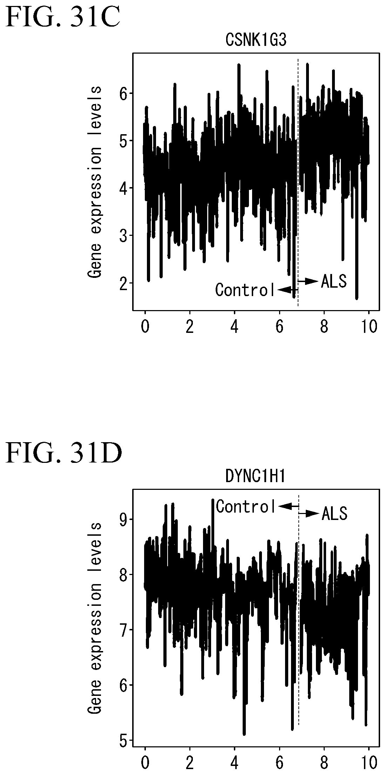

Similarly, the expression amounts of genes extracted from ALS causative genes and ALS related genes were shown in a graph. FIG. 31A is a graph showing expression amounts of SPG11 extracted from ALS causative genes and ALS related genes. FIG. 31B is a graph showing expression amounts of CHMP2B extracted from ALS causative genes and ALS related genes. FIG. 31C is a graph showing expression amounts of CSNK1G3 extracted from ALS causative genes and ALS related genes. FIG. 31D is a graph showing expression amounts of DYNC1H1 extracted from ALS causative genes and ALS related genes. In the drawings, the vertical axis represents gene expression amount, and the horizontal axis represents case. Regarding these genes SPG11, CHMP2B, CSNK1G3, and DYNC1H1, it was not possible to classify the healthy subjects and the ALS patients using each gene alone, and the usefulness of the method of separating healthy subjects and ALS patients using a combination of the three genes PRKAR1A, QPCT, and TMEM71 was demonstrated.

As described above, using a machine learning algorithm called HSIC, which is a high-dimensional nonlinear statistical model, blood molecular biomarkers necessary for digital diagnosis of ALS from actual data were discovered. The identified molecular biomarkers have not been receiving focus for ALS so far. However, it was found that, when expression of these genes was controlled with siRNA, the expression level of TDP-43, which is an important key molecule in ALS, changed, and a possibility of these markers being related to ALS was demonstrated.

The HSIC was used to measure a statistical dependence between two random vectors, the two random vectors were converted into two reproducing kernel Hilbert spaces (RKHS), and the statistical dependence was measured using a Hilbert-Schmidt (HS) operator of these two RKHSs. ALS is a heterogeneous disease exhibiting nonlinear biological phenomena, and its pathogenesis cannot be explained by a single factor. Therefore, this model was applied, and a combination of genes for classifying ALS patients and healthy subjects was searched for using blood sample data. When a nonlinear model was used, a combination of novel genes, PRKAR1A, QPCT, and TMEM71, was successfully found.

The PRKAR1A gene is a gene encoding a serine/threonine kinase, cAMP-dependent protein kinase type I-alpha regulatory subunit, which is a major mediator of cAMP signaling in mammals. Phosphorylation through the cAMP/PKA signal pathway was induced by various physiological ligands within cells, and critically involved in controlling of metabolism, cell proliferation, differentiation, and apoptosis. Loss of one or both allelic genes for this gene caused multiple sclerosis syndromes in humans and embryonic lethal defects in mice. Although the relationship between the PRKAR1A gene and ALS has not been clarified, it has been reported that the PKA activity increased in the spinal cord of ALS patients and the spinal cord of SOD1 mice, and in ALS, the cAMP/PKA synaptic repair promoted activity-dependent neuroprotection of motor neurons. Accordingly, when the expression of the PRKAR1A gene increased, an ALS prevention effect was expected. The Gene ID of the PRKAR1A gene, an example of NCBI Reference Sequence, and the address of the NCBI reference site are as follows.

### PRKAR1A

Gene ID: 5573
NM_001276289.2, NM_212472.1
https://www.ncbi.nlm.nih.gov/gene/5573

The QPTC gene encodes glutaminyl-peptide cyclotransferase. It has been reported that the expression of glutaminyl cyclase increased in the peripheral blood of patients with Alzheimer's disease, and the glutaminyl cyclase inhibitor could be a measure for improving Alzheimer's disease. In addition, it has been reported that QPCT was identified as a therapeutic target for Huntington's disease, and polymorphisms of the QPCT gene were related to susceptibility to schizophrenia. Although the relationship with the pathogenesis of ALS was unclear, the increased QPCT gene expression could be related to a common pathway in neurodegeneration. The Gene ID of the QPTC gene, an example of NCBI Reference Sequence, and the address of the NCBI reference site are as follows.

### OPCT

Gene ID: 25797
NM_012413.4, NM_012413.3
https://www.ncbi.nlm.nih.gov/gene/25797

TMEM71 encodes transmembrane proteins, but its functions have not been clearly elucidated. Knock-out mice exhibited slight hypothyroidism, but no phenotype. The expression of TMEM71 increased in glioblastoma and was related with the immune response, and TMEM71 exhibited a high positive correlation with PD-1 and PD-L1. Knock-out mice exhibited only slight hypothyroidism, but no phenotype. The increased TMEM71 gene expression could be related to the immune response to ALS. The Gene ID of the TMEM71 gene, an example of NCBI Reference Sequence, and the address of the NCBI reference site are as follows.

### TMEM71

Gene ID: 137835
NM_001145153.2, NM_144649.1
https://www.ncbi.nlm.nih.gov/gene/137835

The inventors found a combination of genes for classifying ALS using the nonlinear model HSIC and actual data. This method not only helps with identification of molecular biomarkers for digital diagnosis of ALS but may also lead to a completely new ALS onset mechanism beyond human idea-driven approaches. In addition, this method can be applied not only to ALS but also other multifactorial diseases or sporadic diseases.

### [Reference Signs List]

100 Information processing device
110 Communication interface
120 Input interface
130 Output interface
140 Processor
150 Storage

## Claims

1. An information processing device, comprising
a processor configured to calculate, for combinations of genes included in a gene data set, a scale of dependence of a multifactorial disease or a sporadic disease on causative genes, and the multifactorial disease or the sporadic disease on related genes, and select a combination including a predetermined number of genes from among the data set based on the scale.

2. The information processing device according to claim 1,
wherein the processor distributes, based on an expression amount of a first gene which is a gene derived from a healthy subject, data of the first gene on a certain feature space,
distributes, based on an expression amount of a second gene which is a gene derived from a patient with the multifactorial disease or the sporadic disease, data of the second gene on the feature space,
clusters, based on the expression amount of the first gene, the data of the first gene on the feature space, and
clusters, based on the expression amount of the second gene, the data of the second gene on the feature space.

3. The information processing device according to claim 2,
wherein the processor distributes, in the feature space in which the clusters of the healthy subject and the patient are formed, data of a third gene which is a gene derived from a subject for diagnosis of the multifactorial disease or the sporadic disease,
calculates a distance between the data of the third gene and the cluster on the feature space, and
based on the distance, determines whether the subject will develop the multifactorial disease or the sporadic disease, or determines whether the subject has developed the multifactorial disease or the sporadic disease.

4. The information processing device according to claim 1 or 2,
wherein the multifactorial disease or the sporadic disease includes amyotrophic lateral sclerosis,
wherein the predetermined number is 3, and
wherein the combination including the predetermined number of genes includes at least PRKAR 1 A, QPCT, and TMEM71.

5. The information processing device according to claim 1 or 2,
wherein the processor
performs linear regression analysis and excludes a specific combination including genes whose appearance frequency is equal to or more than a threshold value from a population which is a set of the gene combinations for which the scale is calculated, and
selects the gene combination with the highest scale from the population from which the specific combination is excluded.

6. An information processing method using a computer, comprising:
calculating, for combinations of genes included in a gene data set, a scale of dependence of a multifactorial disease or a sporadic disease on causative genes, and the multifactorial disease or the sporadic disease on related genes; and
selecting a combination including a predetermined number of genes from among the data set based on the scale.

7. The information processing method according to claim 6, further comprising:
distributing, based on an expression amount of a first gene which is a gene derived from a healthy subject, data of the first gene on a certain feature space;
distributing, based on an expression amount of a second gene which is a gene derived from a patient with the multifactorial disease or the sporadic disease, data of the second gene on the feature space;
clustering, based on the expression amount of the first gene, the data of the first gene on the feature space; and
clustering, based on the expression amount of the second gene, the data of the second gene on the feature space.

8. The information processing method according to claim 7, further comprising:
distributing, in the feature space in which the clusters of the healthy subject and the patient are formed, data of a third gene which is a gene derived from a subject for diagnosis of the multifactorial disease or the sporadic disease;
calculating a distance between the data of the third gene and the cluster on the feature space; and
based on the distance, determining whether the subject will develop the multifactorial disease or the sporadic disease, or determining whether the subject has developed the multifactorial disease or the sporadic disease.

9. The information processing method according to claim 7 or 8,
wherein the multifactorial disease or the sporadic disease includes amyotrophic lateral sclerosis,
wherein the predetermined number is 3, and
wherein the combination including the predetermined number of genes includes at least PRKAR1A, QPCT, and TMEM71.

10. The information processing method according to claim 7 or 8, further comprising:
performing linear regression analysis and excluding a specific combination including genes whose appearance frequency is equal to or more than a threshold value from a population which is a set of the gene combinations for which the scale is calculated; and
selecting the gene combination with the highest scale from the population from which the specific combination is excluded.

11. A program causing a computer to execute:
calculating, for combinations of genes included in a gene data set, a scale of dependence of a multifactorial disease or a sporadic disease on causative genes, and the multifactorial disease or the sporadic disease on related genes; and
selecting a combination including a predetermined number of genes from among the data set based on the scale.

12. The program according to claim 11, further comprising:
distributing, based on an expression amount of a first gene which is a gene derived from a healthy subject, data of the first gene on a certain feature space;
distributing, based on an expression amount of a second gene which is a gene derived from a patient with the multifactorial disease or the sporadic disease, data of the second gene on the feature space;
clustering, based on the expression amount of the first gene, the data of the first gene on the feature space; and
clustering, based on the expression amount of the second gene, the data of the second gene on the feature space.

13. The program according to claim 12, further comprising:
distributing, in the feature space in which the clusters of the healthy subject and the patient are formed, data of a third gene which is a gene derived from a subject for diagnosis of the multifactorial disease or the sporadic disease;
calculating a distance between the data of the third gene and the cluster on the feature space; and
based on the distance, determining whether the subject will develop the multifactorial disease or the sporadic disease, or determining whether the subject has developed the multifactorial disease or the sporadic disease.

14. The program according to claim 11 or 12,
wherein the multifactorial disease or the sporadic disease includes amyotrophic lateral sclerosis,
wherein the predetermined number is 3, and
wherein the combination including the predetermined number of genes includes at least PRKAR1A, QPCT, and TMEM71.

15. The program according to claim 11 or 12, further comprising:
performing linear regression analysis and excluding a specific combination including genes whose appearance frequency is equal to or more than a threshold value from a population which is a set of the gene combinations for which the scale is calculated; and
selecting the gene combination with the highest scale from the population from which the specific combination is excluded.
